Europäisches Patentamt

(19) European Patent Office

Office européen des brevets

(11) EP 0 522 920 B1
## (12) FASCICULE DE BREVET EUROPEEN

(45) Date de publication et mention
de la délivrance du brevet:
**26.11.1997 Bulletin 1997/48**

(51) Int. Cl.<sup>6</sup>: **B01D 3/00**

(21) Numéro de dépôt: **92401839.3**

(22) Date de dépôt: **29.06.1992**

(54) **Dispositif de distillation-réaction et son utilisation pour la réalisation de réactions équilibrées**

Destillations-Reaktor und seine Verwendung für Gleichgewichts-Reaktionen

Distillation-reaction device and its use for the realization of balance reactions

(84) Etats contractants désignés:
**DE ES GB IT NL**

(30) Priorité: **09.07.1991 FR 9108716**
**04.02.1992 FR 9201323**

(43) Date de publication de la demande:
**13.01.1993 Bulletin 1993/02**

(73) Titulaire:
**INSTITUT FRANCAIS DU PETROLE**
**92502 Rueil-Malmaison (FR)**

(72) Inventeurs:
• **Marion, Marie-Claire**
**F-69100 Villeurbanne (FR)**
• **Forestière, Alain**
**F-69390 Vernaison (FR)**
• **Delhomme, Henri**
**F-69110 Sainte Foy les Lyon (FR)**

(56) Documents cités:
EP-A- 0 008 860          EP-A- 0 448 884
EP-A- 0 458 472          DE-A- 2 444 888
DE-C- 1 075 613          GB-A- 1 019 236
US-A- 3 629 478          US-A- 4 847 430

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention est relative à un appareil de distillation-réaction et à son utilisation pour la mise en oeuvre de réaction chimique et le fractionnement du mélange de réaction.

Elle est également relative à un procédé de préparation d'un éther par réaction d'oléfines sur des alcools dans lequel on effectue concurremment la réaction et la distillation des produits formés en particulier en vue de séparer ces produits des constituants n'ayant pas réagi.

On sait depuis longtemps que, dans le cas d'une réaction équilibrée, la thermodynamique limite la conversion des réactifs. L'introduction d'un des réactifs en excès permet d'augmenter la conversion des autres réactifs. Mais cette méthode est souvent coûteuse car elle nécessite une installation supplémentaire pour récupérer le réactif introduit en excès.

Un moyen de convertir globalement au-delà de l'équilibre thermodynamique est d'appliquer la méthode dite de distillation-réactive. Cette méthode consiste à effectuer la réaction, habituellement en présence d'un catalyseur, et la distillation dans la même enceinte, pour séparer les produits des autres constituants au fur et à mesure qu'ils se forment. Cette méthode est par exemple utilisée dans le cas de réactions d'éthérification (US-A-3629478, US-A-4847430 et EP-B-8860).

Le brevet US-A-3629478 propose d'utiliser des plateaux de distillation et de ne disposer le catalyseur que dans les descentes de liquide desdits plateaux de distillation, ceci afin d'éviter l'effet perturbateur de la phase vapeur à travers le catalyseur. Toutefois, la présence du catalyseur dans lesdites descentes crée une perte de charge telle que le liquide a tendance à descendre à contre-courant dans les orifices prévus pour le passage de la vapeur sur la table de travail de chaque plateau de distillation. Ainsi, selon cette réalisation, une partie importante du liquide n'entre pas en contact avec le catalyseur ce qui limite l'efficacité, sur le plan de la réaction, de cet appareil de distillation-réaction.

Le brevet US-A-4847430 décrit la mise en oeuvre des zones de réaction-distillation où le lit catalytique alterne avec la zone de distillation. Au travers du lit catalytique, des passages réservés à la phase vapeur évitent le contact gaz-liquide et limitent les problèmes de pertes de charge. Par contre, l'équilibre physique liquide / vapeur n'étant pas réalisé en permanence, il peut y avoir, dans la zone catalytique, épuisement d'un des réactifs. Cela conduit à une perte d'efficacité sur le plan de la réaction, ceci d'autant plus que la colonne réactive travaille dans des conditions proches de l'équilibre chimique.

Le brevet EP-B-8860 propose d'utiliser une colonne de distillation remplie d'un catalyseur approprié pour la préparation du méthyl-tertio-butyl-éther (MTBE), dans laquelle le catalyseur, selon sa mise en forme, assure également au moins en partie la fonction de garnissage pour la distillation, formant ainsi le MTBE et le séparant en même temps des autres constituants présents. Toutefois, la structure globale du garnissage contenant le catalyseur ainsi que son empilement dans la colonne selon la verticale défavorise le contact gaz-liquide et ne présente pas une bonne efficacité sur le plan distillation. Par ailleurs, ce type de remplissage de colonne est coûteux puisqu'il comprend le coût du catalyseur et le coût de fabrication du garnissage catalytique auquel s'ajoute le coût du treillis métallique enroulé autour du matelas catalytique. De plus, lorsqu'il devient nécessaire de changer le catalyseur, on est également obligé de changer le treillis métallique ce qui augmente notablement le coût de cette opération.

Selon la description du brevet EP-B-8860, en particulier selon la revendication 6, le catalyseur est contenu dans des poches textiles supportées par un filet métallique qui leur est intimement associé. Ce filet est, selon la description, obligatoire pour éviter la formation d'un lit très compact qui entraînerait une forte chute de pression (perte de charge) comme le ferait un lit de catalyseur en vrac.

La présente invention vise à remédier aux inconvénients des procédés, et des appareils utilisés pour la mise en oeuvre de ces procédés, décrits dans l'art antérieur, en proposant un appareil permettant de mettre en oeuvre une réaction équilibrée au-delà de l'équilibre thermodynamique dans un appareil comportant une zone de distillation-réaction et permettant d'obtenir une très bonne efficacité à la fois sur le plan de la réaction et sur le plan de la distillation.

A cet effet, l'invention a pour objet un appareil de distillation-réaction tel que défini par la revendication 1

Des modes de réalisations préférés sont définis par les revendications 2 à 6. L'invention concerne aussi l'utilisation d'un tel appareil pour la mise en oeuvre de réactions chimiques et le fractionnement du mélange de réaction et, plus particulièrement, un procédé de préparation d'un éther en présence d'un catalyseur acide.

Dans le cadre de la présente description, le terme support perforé désigne aussi bien une plaque ayant des caractéristiques mécaniques suffisantes pour supporter le poids des éléments de type J et de type K et comportant des perforations laissant passer les divers fluides et suffisamment petites pour retenir lesdits éléments, qu'un ensemble comprenant une plaque et une grille, ladite plaque comportant des perforations laissant passer les divers fluides mais insuffisamment petites pour retenir tous les éléments, et ladite grille comportant des malles laissant passer les divers fluides et suffisamment petites pour retenir le plus petit desdits éléments présents dans la zone de distillation-réaction. Dans ce deuxième cas, l'association grille et plaque possède des caractéristiques mécaniques suffisantes pour supporter le poids des éléments. La grille et la plaque sont faites à partir de matériaux inertes vis-à-vis des divers fluides et solides avec lequel ces matériaux entrent en contact.

Dans la présente description, le terme appareil de distillation-réaction désigne un équipement dans lequel il est possible de réaliser de façon simultanée une réaction chimique et un fractionnement (habituellement multiétagé) et le terme zone de distillation-réaction la zone dans laquelle se déroule de façon concomitante la réaction et le fractionnement.

Dans le cadre de la présente description, les éléments de type J assurent la double fonction réaction et distillation. Selon leur mise en forme, selon leur mode de remplissage, les conteneurs renfermant des particules solides catalytiques permettent, au moins en partie, de gérer le taux de vide défini dans la zone de distillation-réaction.

A l'intérieur du conteneur, le volume Va occupé par l'ensemble des particules solides catalytiques en condition de réaction (c'est-à-dire mouillées par les réactifs), non nul, est inférieur au volume total interne du conteneur Vt. On peut ainsi déterminer un taux de remplissage ($\tau 1$) dudit conteneur : $\tau 1 = Va/Vt$. Ce taux de remplissage est compris entre 0 et 0,9 borne inférieure exclue et borne supérieure incluse et de manière préférée entre 0 et 0,8 borne supérieure incluse, ou de manière encore plus préférée entre 0 et 0,6 borne supérieure incluse. On peut parler également d'un taux de vide ($\tau 2$) à l'intérieur dudit conteneur. Ce taux de vide est défini par la relation : $\tau 2 = (Vt - Va) / Vt$. La somme $\tau 1 + \tau 2$ est dans tous les cas égale à 1. Le taux de remplissage, à l'intérieur de chaque conteneur, est habituellement choisi de manière à ce que les particules solides catalytiques soient mobiles, compte tenu du trafic des fluides, gaz montant et liquide descendant. Ainsi défini, et en condition de fonctionnement, chaque conteneur renfermant des particules catalytiques est considéré comme contenant un lit fluidisé de particules solides catalytiques.

Dans l'appareil de distillation-réaction selon la présente invention, grâce à un empilement aléatoire (c'est-à-dire en vrac) du ou des élément(s) de type J, (c'est-à-dire du ou des conteneur(s) renfermant des particules solides catalytiques) et du ou des élément(s) de type K et grâce à la mobilité des particules solides catalytiques, l'écoulement des flux liquides et gazeux est perturbé en permanence de façon à optimiser les contacts gaz-liquide et liquide-solide (notamment catalyseur). L'équilibre physique liquide-gaz est maintenu en permanence tandis que l'irrigation du catalyseur est réalisée au mieux. Dans la zone de distillation-réaction les fonctions réaction et distillation sont concomitantes ce qui permet une efficacité maximale de la colonne réactive.

Par ailleurs, cette pluralité de lits catalytiques fluidisés, due à la présence du ou des conteneur(s) renfermant des particules solides catalytiques, permet une meilleure évacuation des calories en éliminant les points chauds dûs à l'exothermicité de la réaction et augmente ainsi la durée de vie du catalyseur (souvent sensible à la température).

Enfin, pour une mise en forme donné, la valeur du taux de remplissage de chaque conteneur renfermant des particules solides catalytiques est ajustée de façon à gérer le volume de la zone de distillation-réaction en optimisant les paramètres "dimensionnement de la colonne" et pertes de charge.

Dans le cadre de la présente description, l'expression "sans déformation excessive" signifie que les caractéristiques mécaniques des éléments de type J et de type K, sont telles que, quelle que soit la position de l'un quelconque de ces éléments dans la zone de distillation-réaction, il continue à pouvoir assurer l'ensemble des fonctions qui lui sont propres et en particulier pour le ou les élément(s) de type K à assurer l'existence d'un volume (non nul) exempt de tout solide catalytique et donc de permettre d'obtenir un taux de vide déterminé par rapport à chaque élément et par suite de participer à l'obtention d'un taux de vide global déterminé dans la zone de distillation-réaction.

Dans le cas où au moins un élément de type K est un conteneur vide ou non vide, si V est le volume dudit conteneur après sa fabrication, le volume V1 dudit conteneur au sein de la zone de distillation-réaction est inférieur ou égal à V sans toutefois pouvoir être nul. De préférence on utilise des conteneurs fabriqués de telle façon que, pour chaque conteneur, le volume V1 soit très voisin de V et le plus souvent de telle façon que chaque conteneur ne subisse aucune déformation sous le poids de la charge de l'ensemble des éléments. Ainsi, dans cette forme préférée de réalisation, V1 est compris entre 80 et 100 % et le plus souvent entre 90 et 100 % de la valeur V.

Dans une forme fréquente de réalisation de l'invention, la zone de distillation-réaction comprend une pluralité (c'est-à-dire deux ou plus) d'éléments de type J, et une pluralité d'éléments de type K. Ainsi cette zone comprend une pluralité d'éléments de type K choisis dans le groupe formé par les corps de garnissage de distillation, lesdits corps formant des éléments de type K1, et les conteneurs vides ou non vides ne renfermant pas de particules solides catalytiques, lesdits conteneurs vides ou non vides formant des éléments de type K2. Les éléments de type K1 et ceux de type K2 forment deux sous-groupes distincts, mais ayant les mêmes fonctions, d'éléments de type K.

Les divers conteneurs formant les éléments de type K2 peuvent être identiques entre eux ou différents les uns des autres par exemple par leur taille, leur forme ou par le matériau dont ils sont fabriqués.

Les divers conteneurs de type J renfermant des particules solides catalytiques peuvent être identiques entre eux ou différents les uns des autres par exemple par leur taille, leur forme ou par le matériau dont ils sont fabriqués. Ils peuvent également être différents par leur taux de remplissage $\tau 1$. Le plus souvent, la zone de distillation-réaction comprend plusieurs conteneurs renfermant des particules solides catalytiques identiques entre eux par leur taille, leur forme et par le matériau dont ils sont fabriqués.

La zone de distillation-réaction de l'appareil selon l'invention peut ainsi comprendre, selon une première réalisation, au moins deux éléments de type J et au moins deux éléments de type K1, ou selon une deuxième réalisation au moins deux éléments de type J et au moins deux éléments de type K2, ou selon une autre réalisation au moins deux éléments de type J, au moins un élément de type K1 et au moins un élément de type K2. On peut également envisager d'autres réalisations sans sortir du cadre de la présente invention.

Parmi les éléments de type K2 que l'on utilise on peut distinguer deux catégories différentes : la première constituée par des conteneurs vides et la seconde constituée par des conteneurs non vides contenant un ou plusieurs corps de garnissage.

Selon une forme particulière de mise en oeuvre de l'invention, selon laquelle la zone de distillation-réaction contient des éléments de type J, des éléments de type K1 et des éléments de type K2, le nombre d'éléments de type K2 par rapport au nombre total d'éléments de type K (c'est-à-dire le nombre total d'éléments de type K1 et de type K2) n'est pas critique. On peut ainsi avoir dans la zone de distillation-réaction un seul élément de type K1 ou un seul élément de type K2. On a souvent de 1 à 99 % et de préférence de 1 à 90 % des éléments de type K présents dans la zone de distillation-réaction de l'un ou de l'autre type (K1 ou K2). Dans cette mise en oeuvre particulière, on a ainsi dans la zone de distillation-réaction au moins un élément de type K1 (corps de garnissage) ou au moins un élément de type K2 (conteneur) et souvent de 99 à 1 % et de préférence de 99 à 10 % éléments du même type (K1 ou K2) par rapport au nombre total d'éléments de type K présents dans la zone de distillation-réaction.

Les corps de garnissage utilisés dans la présente invention sont choisis en fonction de l'efficacité nécessaire à la fonction distillation. Par corps de garnissage de distillation, on désigne dans la présente description tous les corps de garnissage bien connus des hommes du métier, tels que par exemple des solides sous forme d'anneaux, d'extrudés polylobés ou de selles. A titre d'exemple non limitatif de corps de garnissage particulier que l'on peut utiliser dans le cadre de la présente invention, on peut citer les anneaux de Raschig, les anneaux de Pall, les anneaux d'Intos, les selles de Berl, les selles Novalox et les selles Intalox. Il est également possible d'utiliser des corps de garnissage géométriquement réguliers tels que par exemple ceux développés il y a 25 ans par la société SULZER ou ceux décrits dans les documents de brevets US-A-3679537, EP-B-70917, EP-A-212202, FR-A-2637059 et FR-A-2637060. On propose aussi comme corps de garnissage des tampons tricotés enroulés de Multiknit ou même des morceaux de grillage. Pour une description de ces corps de garnissage de distillation, on peut également consulter la nouvelle édition en anglais de Ullmann's Encyclopedia of Industrial Chemistry, Volume B3, Unit Operation II, Chapitre 4: Distillation and Rectification, en particulier pages 70 à 92.

Les éléments de type K2 consistant en des conteneurs "non vides" peuvent renfermer, chacun indépendamment les uns des autres, un ou plusieurs corps de garnissage, lesdits corps pouvant être identiques ou différents les uns des autres par leur taille, leur forme ou le matériau dont ils sont fabriqués.

Au sens de la présente description on inclut les cas où le corps de garnissage forme un tout avec le conteneur, c'est-à-dire le cas où ledit corps et l'enveloppe du conteneur sont solidaires l'un de l'autre tel que cela est par exemple le cas lorsque le corps de garnissage est un treillis par exemple métallique ou lorsque l'enveloppe et le corps de garnissage sont fabriqués à partir du même grillage ou tissu.

Les divers éléments de type K (conteneurs vides ou "non vides" ou corps de garnissage) peuvent être identiques entre eux ou différents les uns des autres par exemple par leur taille, leur forme ou par le matériau dont ils sont fabriqués. Le plus souvent, la zone de distillation-réaction comprend plusieurs éléments de type K2 identiques entre eux par leur taille, leur forme et par le matériau dont ils sont fabriqués. Selon une forme préférée de réalisation ces éléments sont des conteneurs vides. Une autre forme de réalisation particulièrement préférée consiste en l'utilisation d'élément de type K1.

Les applications de l'appareil selon la présente invention sont très variées. Cet appareil est aisément modulable en fonction des besoins inhérents à l'application. Ainsi, on sélectionne le catalyseur de type hétérogène en fonction de la réaction concernée.

L'un des intérêts de l'appareil de la présente invention est qu'il permet, sans difficulté particulière, le recyclage du ou des élément(s) de type K. Ce recyclage peut être facilité, en particulier dans le cas où les éléments de type K sont des éléments de type K2, par exemple en utilisant des éléments de type J et de type K2 de tailles ou de formes différentes ou un moyen permettant de faciliter le tri entre les éléments de type J et ceux de type K2. Ces éléments, de type J et de type K2, peuvent être des conteneurs pouvant par exemple avoir, pour chaque type, une enveloppe ayant des caractéristiques facilitant le tri. Sans vouloir y être limité, on peut citer à titre d'exemple le tri magnétique des conteneurs lorsque l'on emploie des éléments de type K comprenant une partie métallique, ou dans le cas des éléments de type K2, comprenant une enveloppe métallique constituée d'un métal M1 et des éléments de type J ne comportant pas de partie métallique ou ne comprenant pas d'enveloppe métallique constituée à partir du métal M1, mais pouvant être constituée à partir d'un métal M2 ayant des propriétés magnétiques différentes de celles du métal M1.

De préférence, l'enveloppe externe fermée, formant tout conteneur de type K2 renfermant ou non un ou des corps de garnissage, est perméable aux fluides, c'est-à-dire aux liquides et aux gaz et imperméable aux autres éléments présents, c'est-à-dire aux autres conteneurs, au(x) corps de garnissage renfermé(s) dans ledit conteneur lorsqu'il n'est pas vide et au(x) corps de garnissage éventuel(s) contenu(s) dans la zone de distillation-réaction. Cette enveloppe empêche ainsi dans le cas d'un conteneur de type K2 non vide la sortie du ou des corps de garnissage dudit conteneur tout en autorisant le passage des liquides et des gaz à travers ledit conteneur. Les corps de garnissage contenus dans des conteneurs conservent ainsi toute leur efficacité en distillation.

Selon la présente invention, l'enveloppe externe fermée formant tout conteneur de type J, renfermant des particules solides catalytiques, est perméable aux fluides c'est-à-dire aux liquides et aux gaz, et imperméable aux particules solides catalytiques (catalyseur), au(x) corps de garnissage contenu(s) dans la zone de distillation-réaction et aux autres conteneurs présents dans la zone de distillation-réaction. Cette enveloppe empêche ainsi la sortie des particules solides catalytiques.

L'enveloppe de tout conteneur de type J est habituellement réalisée à partir d'un matériau solide qui peut être un matériau perméable ou poreux ou même un matériau imperméable dans lequel on a prévu des ouvertures (ou pores) ayant une taille suffisamment petite pour maintenir les particules solides catalytiques à l'intérieur du conteneur.

L'enveloppe des conteneurs formant les éléments de type K2 est habituellement réalisée à partir d'un matériau solide qui peut être identique ou différent de celui choisi pour réaliser les conteneurs formant les éléments de type J. On peut ainsi employer un matériau perméable ou poreux ou même un matériau imperméable dans lequel on a prévu des ouvertures (ou pores) ayant une taille suffisamment petite pour éviter dans le cas des conteneurs formant les éléments de type K2 que les autres éléments ne pénètrent dans ces conteneurs, et de plus dans le cas des conteneurs formant les éléments de type K2, non vides, pour maintenir le (ou les) corps de garnissage de distillation à l'intérieur du conteneur. Dans les deux cas les tailles des ouvertures des conteneurs formant les éléments de type K2 sont suffisantes pour permettre le passage des fluides à travers ledit conteneur.

Il est possible de mieux préciser, notamment dans le cas des conteneurs formant les éléments de type J, les caractéristiques géométriques de ces ouvertures, sans que cela ne soit considéré comme une limitation de la présente invention. Dans ce cas, si la plus petite dimension de la plus petite particule solide catalytique est égale à "n" mètre (m), alors la plus grande dimension des ouvertures permettant le passage des fluides est le plus souvent inférieure ou égale à "0,9xn" m et de préférence inférieure ou égale à "0,5xn" m. Il n'y a en principe pas de limite pour les dimensions des particules solides catalytiques ou grains de catalyseur et la limite inférieure de la plus petite dimension des ouvertures est égale à la dimension minimum permettant le passage des fluides et en particulier du liquide. Dans la grande majorité des cas, la zone de distillation-réaction contient des conteneurs formant les éléments de type J renfermant des catalyseurs dont la granulométrie peut être comprise entre $5 \times 10^{-6}$ m et $2 \times 10^{-1}$ m. A titre d'exemple non limitatif, dans le cas de l'utilisation d'un catalyseur acide pour la synthèse d'un éther à partir d'une oléfine et d'un alcool, les grains de catalyseur ont le plus souvent une granulométrie comprise entre $1 \times 10^{-4}$ m et $2 \times 10^{-2}$ m. Dans la forme de réalisation la plus fréquente les dimensions des ouvertures sont identiques pour les conteneurs de chaque type présents dans la zone de distillation-réaction et sensiblement identiques d'un conteneur à l'autre.

Comme exemple de matériau utilisable pour former l'enveloppe des conteneurs, on peut citer les matériaux tissés ou les matériaux non-tissés. La matière utilisable pour former l'enveloppe des conteneurs peut être d'origine naturelle telle que minérale, végétale ou animale, ou encore d'origine synthétique. Comme matière on peut citer, à titre d'exemple non limitatif, le polypropylène, les polyesters, les polyamides, l'aluminium, le cuivre, le titane, le nickel, le platine, l'acier inoxydable ou un grillage ou treillis métallique, les dimensions des ouvertures ou mailles de ce grillage ou treillis étant telles que définies ci-avant.

La matière choisie doit être physiquement et chimiquement inerte vis-à-vis des fluides et des solides avec lesquels elle entre en contact.

Lorsque la matière choisie pour former l'enveloppe des conteneurs, en particulier pour ceux formant les éléments de type J mais également pour ceux formant des éléments de type K2 consistant en des conteneurs vides, n'a pas de caractéristiques mécaniques suffisantes pour permettre d'obtenir des conteneurs capables de résister au poids de l'ensemble des éléments présents dans la zone de distillation-réaction, on inclut des moyens de renforcement mécanique tels que par exemple des tiges d'acier ou tout autre moyen connu des hommes du métier permettant de fabriquer des conteneurs ayant les caractéristiques mécaniques souhaitées.

Dans le cas des conteneurs formant les éléments de type K2 non vides, selon le (ou les) corps de distillation sélectionné(s), on choisit de préférence le matériau constituant l'enveloppe du conteneur de manière à ce que l'association des deux permette d'obtenir un ensemble corps de garnissage-enveloppe du conteneur ayant une certaine rigidité, c'est-à-dire doué d'une résistance à l'écrasement suffisante pour qu'un conteneur situé à la base de la zone de distillation-réaction ne soit pas écrasé par le poids des éléments. Ainsi si le corps de garnissage de distillation est rigide, l'enveloppe peut être réalisée à partir d'un matériau souple (par opposition à rigide). Au contraire, si le corps de garnissage de distillation est peu rigide, l'enveloppe est de préférence réalisée à partir d'un matériau rigide.

Dans le cas des conteneurs formant les éléments de type J, il est souvent préférable d'employer soit un matériau relativement rigide pour l'enveloppe du conteneur, soit d'utiliser des conteneurs comportant des moyens de renfort mécaniques. Selon les caractéristiques mécaniques du catalyseur, on choisit de préférence le matériau constituant l'enveloppe du conteneur de manière à ce que l'association des deux permette d'obtenir un ensemble catalyseur - enveloppe du conteneur ayant une certaine rigidité, c'est-à-dire doué d'une résistance à l'écrasement suffisante pour qu'un conteneur situé à la base de la zone de distillation-réaction ne soit pas écrasé par le poids de l'ensemble des éléments de ladite zone. Ainsi si le catalyseur est peu friable et/ou non cassant l'enveloppe peut être réalisée à partir d'un matériau souple (par opposition à rigide). Au contraire, si le catalyseur est relativement friable et/ou cassant, l'enveloppe est de préférence réalisée à partir d'un matériau rigide. Il est souvent préférable, pour éviter tous risques de formation de fines par effritement du catalyseur, d'employer soit un matériau relativement rigide pour l'enveloppe du conteneur, soit d'utiliser des conteneurs comportant des moyens de renfort mécaniques.

L'invention permet une bonne maîtrise du taux de vide dans la zone de distillation-réaction, une bonne maîtrise du taux de remplissage de cette zone et au cours de l'utilisation de l'appareil et une bonne maîtrise des pertes de charge. Le taux de vide à l'intérieur de la zone de distillation-réaction peut être fixé à une valeur préalablement choisie qui est obtenue par exemple en choisissant le taux de remplissage de chacun des conteneurs renfermant des particules catalytiques et le nombre d'éléments de type K, c'est-à-dire de type K1 et/ou de type K2, inclus dans la zone de distillation-réaction. Le taux de vide varie également en fonction du mode de remplissage des conteneurs non vides, formant les éléments de type K2, et de la qualité et de la quantité de corps de garnissage de distillation renfermée dans chaque conteneur K2 non vide.

Dans une forme de réalisation particulière de l'invention, chaque conteneur ou un certain nombre d'entre eux, comporte au moins un moyen, et de préférence au moins deux moyens, ayant une fonction de renforcement mécanique dudit conteneur et pouvant avoir une fonction secondaire de maintien d'un espace minimum entre ledit conteneur et le conteneur le plus proche en contact avec l'extrémité dudit moyen et/ou de l'une des surfaces solides la plus proche en contact avec l'extrémité dudit moyen. Ce ou ces moyen(s) peuvent être un ou plusieurs bourrelet(s) ou ailette(s) rigide(s). Ils permettent de réaliser des conteneurs ayant une enveloppe en matériau relativement souple tout en ayant les caractéristiques mécaniques souhaitées. Ils peuvent en outre permettre de réguler le taux de vide de la zone de distillation-réaction et permettent également une bonne maîtrise des pertes de charges. Ils peuvent de plus faciliter le tri, par exemple magnétique, des conteneurs par exemple de ceux de type K2 ne renfermant pas de particules solides catalytiques.

Le nombre, la dimension et la qualité des ailettes permettent de déterminer les caractéristiques mécaniques globales des conteneurs, et en outre d'agir sur le taux de vide et le taux de remplissage de la zone de distillation-réaction. De la même façon, ces ailettes peuvent permettre de définir et de moduler la perte de charge. L'utilisation de conteneurs comportant des ailettes est, par rapport à celle de l'utilisation de conteneurs vides de type K2, une autre manière, complémentaire ou non, d'agir sur le taux de vide de la zone de distillation-réaction. En effet, plus la dimension et le nombre d'ailettes sont importants, plus la place disponible entre les conteneurs des divers types est importante et plus le taux de vide est important.

Ces ailettes présentent de préférence une rigidité importante. Elles doivent être douées d'une résistance à l'écrasement, de façon à assurer leur fonction de renfort mécanique et éventuellement d'espacement du ou des conteneur(s) entre eux et aussi avec les parois de l'appareil et également avec les corps de garnissage éventuellement présents dans la zone de distillation-réaction. Ces ailettes peuvent comporter une plaque, un renfort ou une tige de renforcement ou plus simplement résulter de la soudure ou du collage des extrémités de l'enveloppe.

La dimension de l'ailette est liée à la dimension du conteneur. Sa longueur (L) est le plus souvent comprise entre environ "0,01xp" m et "10xp" m et de préférence entre environ "0,05xp" m et "1xp" m, si "p" désigne la plus grande dimension du conteneur. L'épaisseur des ailettes peut varier dans de larges proportions ; elle est le plus souvent comprise entre $1 \times 10^{-4}$ m et $5 \times 10^{-2}$ m. Le nombre d'ailettes n'est pas limité. Il peut par exemple être de 1 à 20 et est le plus souvent de 2 à 10. Dans un exemple de réalisation de fabrication de conteneurs comportant des ailettes, de mise en oeuvre particulièrement facile, ceux-ci comportent deux ailettes.

La plus grande dimension des éléments présents dans la zone de distillation-réaction, et en particulier des conteneurs, est habituellement inférieure à 0,1 fois, et de préférence inférieure à 0,07 fois le diamètre de la zone de distillation-réaction dans le cas le plus fréquent où celle-ci a une section sensiblement circulaire. Dans ce cas, il est cependant possible, sans sortir du cadre de la présente invention, d'utiliser des éléments dont la plus grande dimension peut avoir une valeur supérieure à 0,1 fois le diamètre de la zone de distillation-réaction et au plus égale au diamètre de ladite zone.

Le plus souvent, les conteneurs vides sont réalisés à l'aide d'un matériau présentant des caractéristiques mécaniques suffisantes pour qu'il ne soit pas indispensable d'inclure des moyens mécaniques de renforcement pour obtenir les caractéristiques mécaniques souhaitées pour le conteneur ainsi fabriqué. Dans ce cas, on choisit par exemple des matériaux métalliques tels que l'aluminium, l'acier inoxydable, le plus souvent sous forme de grillage ou treillis métallique. La forme géométrique des conteneurs n'est pas critique ; ils peuvent être sensiblement tétraédriques, sensiblement octaédriques, sensiblement sphériques ou de tout autre forme.

Selon une forme avantageuse de réalisation de l'invention, les éléments sont disposés entre ledit support perforé et un dispositif perforé similaire permettant le passage des fluides et dont les perforations sont suffisamment petites pour retenir lesdits éléments entre ledit support et ledit dispositif. Cette forme de réalisation permet de diminuer la mobilité des éléments, en particulier lorsque ceux-ci sont de dimensions relativement petites, et d'éviter largement d'éventuelles ségrégations entre les divers éléments de type J et de type K. Les éléments occupent généralement au moins une partie du volume de la zone de distillation-réaction , c'est-à-dire entre 1 et 100 % et de préférence 100 % dudit volume.

Dans une forme particulièrement avantageuse de mise en oeuvre de l'invention, les éléments sont disposés en vrac dans des sacs de préférence souples, ou dans des paniers de préférence rigides, avant d'être introduits dans la zone de distillation-réaction. Ces sacs ou paniers sont fabriqués à partir de matériau sous forme de grillage ou de treillis dont les mailles sont suffisamment petites pour retenir les divers éléments. De préférence les mailles des sacs ou des paniers sont les plus grandes possibles de manière à limiter au maximum les problèmes de perte de charge. Ces sacs et/ou ces paniers ont de préférence une taille permettant de les introduire facilement par un trou d'homme dans la zone de distillation-réaction. Dans le cas de l'utilisation de paniers, il est avantageux d'employer des paniers qui, mis côte à côte, occupent toute la surface de la zone de distillation-réaction. Ces sacs et/ou ces paniers sont le plus souvent empilés en couches successives sur toute la hauteur de la zone de distillation-réaction. Dans le cas de paniers de section sensiblement rectangulaire l'empilement est de préférence réalisé de manière à ce que la direction définie par le grand côté du rectangle soit d'une couche à l'autre sensiblement orthogonale. Dans le cas d'une zone de distillation-réaction de section sensiblement circulaire on peut utiliser des paniers de forme adaptée, de manière à occuper le maximum de surface de la section.

L'appareil de distillation-réaction selon l'invention comprend de préférence au moins deux zones de distillation-réaction, contenant chacune au moins un élément de type J et au moins un élément de type K, lesdites zones n'étant pas en contact l'une avec l'autre, c'est-à-dire que l'appareil comporte alors entre les zones successives de distillation-réaction des zones ou espaces vides et/ou comprenant des plateaux ou systèmes de redistribution de liquide et/ou des zones de distillation (comprenant par exemple des plateaux à calottes, des plateaux à clapets etc.) et/ou des zones comprenant des corps de garnissage, lesdits corps étant éventuellement renfermés dans des conteneurs, et/ou des conteneurs vides.

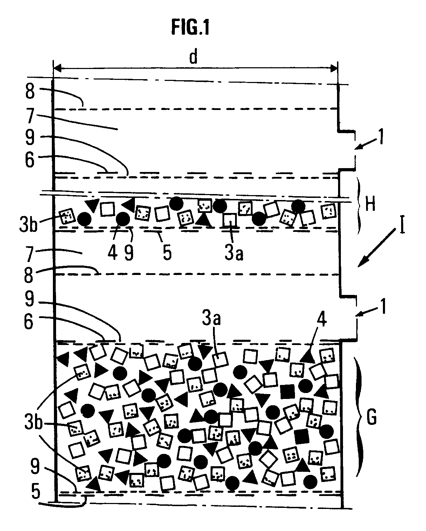

Les figures 1, 2A, 2B, 2C, 2D, 2E, 2F, 3, 4, 5, 5A, 5B, 6A et 6B illustrent l'invention sans en limiter la portée. Sur ces figures les organes similaires sont désignés par les même chiffres et lettres de référence.

La figure 1 représente schématiquement une partie d'un appareil (I) de distillation-réaction selon l'invention, comprenant selon la forme avantageuse de réalisation schématisée sur cette figure, deux zones (H) et (G) de distillation-réaction, une zone (7) renfermant un plateau (8) de redistribution de liquide et séparant les zones (H) et (G) et également une zone (7) surmontant la zone (G). Cet appareil a la forme d'une colonne sensiblement cylindrique et verticale de diamètre (d) sensiblement constant sur toute la hauteur de la colonne. Chacune des zones de distillation-réaction (H) et (G) comprend en vrac, sur un support perforé (5) (laissant passer le flux gazeux ascendant et le flux liquide descendant et retenant les éléments) des éléments (3a, 3b et 4) dans le cas de la réalisation schématisée sur la figure 1. Les éléments de type J sont des conteneurs (3b) renfermant des particules solides catalytiques (2) en grain. Les conteneurs (3a) formant des éléments de type K2 sont des conteneurs vides et les corps (4) de garnissage de distillation forment les éléments de type K1. Dans le cas schématisé sur la figure 1, les éléments de type K1 sont des corps de garnissage qui ne sont pas tous identiques. La zone de distillation-réaction, comprenant les conteneurs (3a) et (3b) et les corps de garnissage (4) , est surmontée par une plaque perforée (6) fixée dans la colonne par des moyens non représentés sur la figure 1. Les éléments sont introduits dans la colonne par des trous d'hommes (1) et la plaque (6) (ou les composants nécessaires à sa fabrication) est installée ensuite dans la colonne de manière à limiter au maximum les mouvements possibles desdits éléments. Les supports (5) et les plaques (6) sont maintenus en place dans la colonne par tout moyen adéquat tel que par exemple des taquets non représentés sur la figure 1 ou par soudure.

Comme cela est représenté sur la figure 1, les supports (5) peuvent être associés à un tissu (9) (ou à une grille fine présentant des ouvertures plus petites que celles du support (5) mais de taille suffisante pour laisser passer le flux gazeux ascendant et le flux liquide descendant), qui est fixé par tout moyen adéquat au support (5) ou de préférence à la paroi de l'appareil. Le tissu (ou la grille) est généralement fixé au-dessus du support (5). Dans le cas de l'utilisation d'une grille métallique celle-ci peut par exemple être fixée par soudure au niveau des parois de l'appareil. L'emploi d'un tissu (ou d'une grille) est habituellement conseillé uniquement si les ouvertures du support (5) sont trop grandes pour retenir les éléments employés. La même disposition peut être prise pour les plaques (6) dans le cas où leurs ouvertures sont trop grandes pour retenir les éléments employés, le tissu (9) (ou la grille) peut dans ce cas être fixé de préférence au-dessous des plaques (6).

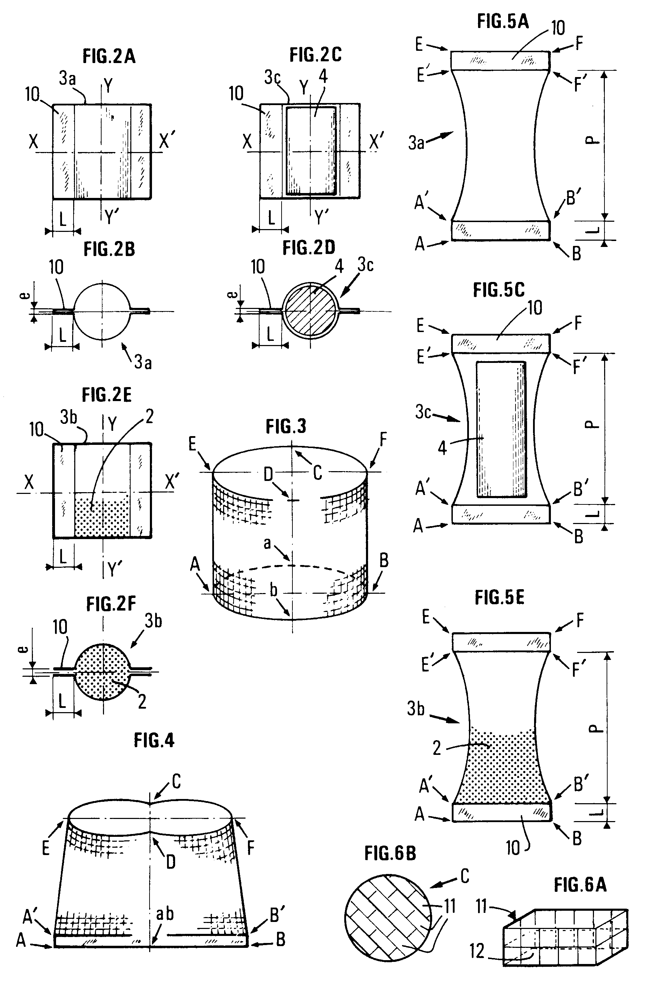

La figure 2A est une coupe vue de face d'un conteneur (3a) vide. La figure 2C est une coupe vue de face d'un conteneur (3c) renfermant un corps de garnissage (4) et la figure 2E celle d'un conteneur (3b) renfermant des particules solides catalytiques (2).

Les figures 2B 2D et 2F sont chacune une vue en coupe, selon un plan perpendiculaire à l'axe yy′ et passant par l'axe xx′, du conteneur représenté respectivement sur la figure 2A, 2C et 2E. Ces conteneurs comportent deux ailettes (10) de longueur (L) (figures 2A, 2C et 2E) et d'épaisseur (e) (figures 2B, 2D et 2F).

Les figures 3, 4, 5A, 5C et 5E illustrent les étapes de fabrication d'un conteneur, vide ou renfermant des particules catalytiques ou un corps de garnissage de distillation, relatif à l'invention. Dans le cas schématisé sur les figures 2C, 2D et 5C, le corps de garnissage de distillation est de forme cylindrique (anneau de Pall ou anneau de Raschig par exemple). Pour la fabrication des conteneurs, on part d'une portion de tube en matière poreuse ou perméable, par exemple en tissu métallique. La section droite de ce tube peut être circulaire, ovale ou avoir tout autre forme ; le tube est donc qualifié ci-après de "cylindrique ou équivalent". Dans cette définition on inclut aussi le cas où la portion de tube ne serait pas exactement cylindrique ou équivalente, c'est-à -dire que l'arête du cylindre n'aurait pas la même longueur en tous points. Ceci peut être réalisé par exemple en tranchant obliquement et non perpendiculairement un tube de plus grande longueur lorsqu'on veut former la portion de tube nécessaire à la fabrication du conteneur. Pour la fabrication des conteneurs formant les éléments de type K2 non vides, le diamètre (moyen) du tube utilisé est le plus souvent au moins supérieur à 1,1 fois le diamètre du corps de garnissage de distillation. Les bases supérieure et inférieure sont initialement ouvertes (Figure 3). On rapproche les deux demi-circonférences (a) et (b) définies par leurs extrémités communes A et B jusqu'à les mettre en contact et on fixe ensemble, par exemple par soudage, ces deux demi-circonférences, qui forment alors un bourrelet (une ailette) sensiblement linéaire ABB′A′ (Figure 4).

Dans les cas des conteneurs formant les éléments de type J et de type K2 non vides, on introduit soit les particules catalytiques (2), soit le ou les corps (4) de garnissage de distillation (Figures 5E et 5C) dans le sac dont le fond a été fermé comme expliqué plus haut, puis on répète l'opération de rapprochement et fixation cette fois avec les demi-circonférences définies par leurs extrémités communes C et D, ou E et F ou tout couple de points symétriques par rapport au centre de la section du tube. Aussi bien dans le cas des conteneurs vides que dans celui des conteneurs non vides, après fixation, par exemple par soudage des demi-circonférences, on a formé un bourrelet (une ailette) sensiblement linéaire EE′FF′ (Figures 5A, 5C et 5E). Les conteneurs ainsi obtenus possèdent deux ailettes dont la taille est modulable. La longueur L de chacune de ces ailettes est égale respectivement à AA′ ou BB′ et à EE′ ou FF′. Cette longueur est fonction de la largeur de la soudure. Son épaisseur est environ 2 fois supérieure à l'épaisseur du matériau constituant le tube. La dimension de ces ailettes selon l'axe sensiblement parallèle à l'axe défini par les extrémités AB et EF est sensiblement égale à la longueur de chaque demi-circonférence qui lui a donné naissance. Dans le cas de réalisation schématisée sur ces figures la plus grande dimension "p" (figure 5C) du conteneur est sensiblement égale à la plus grande dimension du corps de garnissage renfermé dans ledit conteneur.

La figure 6A représente schématiquement en perspective un panier (11) formé à partir de fil d'acier rigide comportant des mailles (12) dont les dimensions sont suffisamment petites pour retenir les éléments et de préférence les plus grandes possibles et juste assez petites pour retenir les éléments. Ces paniers sont remplis avec les éléments des divers types puis introduits dans la colonne I dans la zone de distillation-réactive. La figure 6B représente schématiquement, en coupe suivant un plan horizontal, la répartition des paniers dans la colonne I. La forme de chaque panier est choisie en fonction de sa position dans la colonne de manière à ce que l'ensemble des paniers occupe le maximum de surface.

La présente invention a également pour objet l'utilisation de l'appareil décrit ci-avant pour la mise en oeuvre de réactions chimiques et le fractionnement du mélange de réaction. En particulier l'invention concerne l'utilisation de l'appareil décrit ci-avant pour la production d'éthers par réaction d'oléfines, ayant de 3 à 8 atomes de carbone par molécule, avec des alcools ayant de 1 à 6 atomes de carbone par molécule. A titre d'exemple particulier des oléfines que l'on peut utiliser, on peut citer le propène, l'isobutène, ou les autres butènes isomères et l'isoamylène ou les autres pentènes isomères. De même à titre d'exemple d'alcools que l'on peut employer on peut citer le méthanol, l'éthanol, le n-propanol, l'isopropanol et les butanols. Les éthers les plus souvent fabriqués industriellement de nos jours comprennent le méthyltertiobutyléther (MTBE), l'éthyltertiobutyléther (ETBE), l'isopropyltertiobutyléther (IPTBE), le méthyltertioamyléther (TAME) et l'éthyltertioamyléther (ETAE).

L'invention concerne aussi le procédé de préparation d'un éther par réaction d'oléfines, ayant de 3 à 8 atomes de carbone par molécule, avec des alcools ayant de 1 à 6 atomes de carbone par molécule, en présence d'un catalyseur acide sous forme de particules solides, dans lequel on effectue concurremment la réaction et la séparation par distillation des produits formés au cours de ladite réaction dans un appareil tel que décrit ci-avant et dans lequel on récupère en continu l'éther formé. Le catalyseur acide que l'on utilise le plus fréquemment pour effectuer cette réaction est une résine échangeuse d'ions sous forme acide telle que par exemple une résine sulfonée (en particulier une résine polystyrène-divinylbenzène sulfonée comme par exemple l'Amberlyst 15 fabriqué par Rohm et Haas). On utilise de préférence des oléfines ayant de 4 à 6 atomes de carbone par molécule et de préférence des alcools ayant de 1 à 4 atomes de carbone par molécule. Le plus souvent les oléfines utilisées sont des oléfines tertiaires.

Les conditions de préparation d'éther à partir d'au moins une oléfine et d'au moins un alcool sont des conditions classiques bien connues de l'homme du métier. A titre indicatif on maintient en général un rapport de reflux par rapport au distillat (c'est-à -dire un rapport entre le volume de liquide reflué et le volume de liquide soutiré) comprise entre 0,1:1 et 20:1, de préférence comprise entre 0,5:1 et 5:1. On opère le plus souvent, à l'intérieur de l'appareil (I), dans un domaine de pression et de température assez large : par exemple, de 100 à 3000 kilopascals (kPa), de préférence de 200 à 2000 kPa pour la pression et de 10 à 200°C, de préférence de 40 à 120°C pour la température (dans tout l'appareil). Dans chaque zone de distillation-réaction les conteneurs occupent toute la section sensiblement circulaire de la zone de distillation-réaction. Les particules solides renfermées dans les conteneurs formant les éléments de type J peuvent être conditionnées sous toute forme adéquate, notamment sous forme sensiblement cylindrique ou sous forme sensiblement sphérique.

## Exemple 1

La détermination de la hauteur équivalente en plateaux théoriques (HEPT) permet de caractériser l'efficacité d'un appareil de distillation-réaction sur le plan de la distillation. Dans cet exemple, les HEPT ont été déterminées par la méthode dite de Mac Cabe et Thiele en distillant, à reflux total, un mélange binaire méthanol-éthanol. A l'équilibre, l'analyse (indice de réfraction) du distillat (échantillon prélevé en haut de colonne) et du résidu (échantillon prélevé dans le bouilleur) permet d'obtenir graphiquement, à partir du diagramme d'équilibre liquide/vapeur, le nombre de plateaux théoriques de la colonne. Les expériences ont été faites dans un appareil de laboratoire, ayant la forme d'une colonne de section cylindrique de diamètre intérieur de 100 millimètre (mm), disposée sur un ballon (bouilleur) de 10 litres, équipée d'un réfrigérant et d'un système de reflux. L'appareil est muni de deux prises de température en haut et en bas de colonne. Ces expériences sont faites à reflux total. A l'équilibre thermique, on prélève un échantillon dans le ballon (Résidu ou Soutirat), un échantillon en haut de colonne au niveau du reflux (Distillat). L'appareil "C1" est rempli sur une hauteur de 1000 mm avec le garnissage pour colonne réactive décrit dans le texte du brevet EP-B- 8860 et schématisé sur les figures 2 et 3 de ce brevet. Ce garnissage repose sur un support perforé dont les ouvertures circulaires ont un diamètre de 9 mm. Dans un appareil "C2" identique à l'appareil "C1" on introduit en vrac des conteneurs vides, des anneaux de Pall, et des conteneurs renfermant des particules de catalyseur en grain en quantité telle que dans les conditions de la réaction ces particules occupent un volume sensiblement égal à la moitié du volume du conteneur les renfermant. La plus grande dimension de ces conteneurs est de 15 mm et la plus petite de 12 mm. Les anneaux de Pall ont un diamètre de 15 mm et une hauteur de 15 mm. L'enveloppe des conteneurs est en polypropylène tissé ayant des mailles carrées de 0,3 mm de côté. Ces conteneurs comportent tous une armature de renfort en acier inoxydable. La hauteur totale du lit formé par les conteneurs dans la colonne est de 1000 mm. Dans chacune des deux expériences, la quantité de catalyseur dans la colonne, en poids de catalyseur sec, est identique. Le catalyseur utilisé est la résine sulfonée vendue par la société Rohm & Haas sous le nom commercial Amberlyst 15. Dans le cas de l'appareil "C1" on trouve un HEPT de 0,4 m et dans le cas de l'appareil "C2" de 0,30 m. L'appareil selon l'invention permet donc un gain important en efficacité de distillation puisque on a un gain d'environ 25 % sur la hauteur d'un plateau théorique.

## Exemple 2

Pour simuler le réacteur de finition d'une unité industrielle, on réalise la synthèse du MTBE dans une colonne réactive expérimentale. Elle est alimentée par une charge renfermant du méthanol et un mélange de butènes et butanes contenant environ 25 % d'isobutène déjà converti à 80 % en MTBE. La première expérience (I) est menée avec l'appareil "C1" décrit dans l'exemple 1. Dans la deuxième expérience (II), qui illustre la présente invention, on utilise l'appareil "C2" décrit dans l'exemple 1. En opérant à une pression de l'ordre de 0,5 Mégapascal (MPa), à une température comprise entre 60 et 80 °C et en maintenant un rapport de reflux de l'ordre de 1/1, on convertit en MTBE environ 60 % de l'isobutène résiduel dans la première expérience contre environ 90 % dans la deuxième expérience.

L'appareil selon la présente invention permet donc d'obtenir une conversion nettement supérieure à celle obtenue avec un appareil réalisé selon l'enseignement du brevet EP-B-8860.

## Revendications

1. Appareil de distillation-réaction comportant au moins une zone de distillation-réaction, ladite zone contenant, sur un support perforé permettant le passage des fluides, une pluralité d'éléments de type J comprenant un conteneur renfermant une quantité de particules solides catalytiques telle que le taux de remplissage $Z_1$ du conteneur étant le rapport entre le volume $V_a$ de ces particules, mesuré après leur mise en contact avec la charge dans les conditions de la réaction, et le volume interne $V_t$ dudit conteneur, est compris entre 0 et 0,9 borne inférieure exclue et borne supérieure incluse, ledit conteneur ayant une enveloppe externe perméable aux fluides et imperméable auxdites particules solides catalytiques, et une pluralité d'éléments de type K ayant au moins la double fonction d'assurer un taux de vide déterminé dans la zone de distillation-réaction et d'assurer un effet de distillation, l'élément de type K étant choisi dans le groupe formé par les corps de garnissage de distillation formant des éléments du type K1, et les conteneurs vides ou les conteneurs non vides contenant un ou plusieurs corps de garnissage, ou non vides formant des éléments de type K2, lesdits éléments de type J et de type K formant un empilement aléatoire, en vrac, permettant l'écoulement des fluides et ayant des caractéristiques mécaniques suffisantes pour résister, sans déformation excessive, à la charge de l'ensemble des éléments de type J et de type K présents dans ladite zone de distillation-réaction, et les perforations dudit support perforé étant suffisamment petites pour retenir lesdits éléments de type J et de type K.

2. Appareil selon la revendication 1 dans lequel la zone de distillation-réaction contient l'une au moins des combinaisons : au moins deux éléments de type J et au moins deux éléments de type K1 ; au moins deux éléments de type J et au moins deux éléments de type K2 ; au moins deux éléments de type J, au moins un élément de type K1 et au moins un élément de type K2.

3. Appareil selon l'une des revendications 1 ou 2 comprenant de 1 à 99 % des éléments de type K dans la zone de distrillation-réaction de l'un ou de l'autre type, K1 ou K2.

4. Appareil selon l'une des revendications 1 à 3 dans lequel au moins un conteneur choisi dans la groupe formé parmi les éléments de type J et les éléments de type K comporte au moins un moyen ayant la double fonction de renforcement mécanique dudit conteneur et de maintien d'un espace minimum entre ledit conteneur et l'un au moins des éléments suivants : le conteneur la plus proche on contact avec l'extrémité dudit moyen, l'une des surfaces solides la plus proche en contact avec l'extrémité dudit moyen.

5. Appareil selon l'une des revendications 1 à 4 dans lequel les éléments de type J et les éléments de type K occupent au moins une partie du volume de la zone de distillation-réaction compris entre ledit support perforé et un dispositif perforé similaire permettant le passage des fluides et dont les perforations sont suffisamment petites pour retenir lesdits éléments entre ledit support et ledit dispositif.

6. Appareil selon l'une des revendications 1 à 5 dans lequel les éléments de type K comportent des moyens permettant d'effectuer leur séparation des éléments de type J par utilisation de moyens magnétiques.

7. Utilisation d'un appareil selon l'une des revendications 1 à 6 pour la mise en oeuvre de réactions chimiques et le fractionnement du mélange de réaction.

8. Utilisation d'un appareil selon l'une des revendications 1 à 7 pour la production d'éthers par réaction d'oléfines, ayant de 3 à 8 atomes de carbone par molécule, avec des alcools ayant de 1 à 6 atomes de carbone par molécule.

9. Procédé de préparation d'un éther par réaction d'oléfines, ayant de 3 à 8 atomes de carbone par molécule, avec des alcools ayant de 1 à 6 atomes de carbone par molécule, en présence d'un catalyseur acide sous forme de particules solides, selon lequel on effectue concurremment la réaction et la distillation des produits formés au cours de ladite réaction dans un appareil selon l'une des revendications 1 à 6 et dans lequel on récupère en continu l'éther formé.

**Claims**

1. Distillation-reaction apparatus having at least one distillation-reaction zone, said zone containing, on a perforated support permitting the passage of fluids, several elements of type J comprising a container containing a quantity of catalytic solid particles such that the volume Va of said particles measured after their contacting with the charge under the conditions of the reaction, and the internal volume Vt of said container, is comprised between 0 and 0,9, the lower limit excluded and the upper limit included, said container having an outer envelope permeable to fluids and impermeable to the solid catalytic particles, and several elements of type K having at least the double function of ensuring a predetermined vacuum level in the distillation-reaction zone and ensuring a distillation effect, the element of type K is chosen from within the group formed by distillation packing materials forming the elements of type K1 and empty containers or non-empty containers not containing solid catalytic particles, said empty or non-empty containers forming elements of type K2, said elements of types J and K forming a random stock, in bulk, permitting the passage of the fluids having adequate mechanical characteristics to withstand, without excessive deformation, the load of all the elements of types J and K present in said distillation-reaction zone, and the perforations of said perforated support are sufficiently small to retain said elements of types J and K.

2. Apparatus according to claim 1, wherein the distillation-reaction zone contains at least one of the following combinations : at least two elements of type J and at least two elements of type K1, at least two elements of type J and at least two elements of type K2, at least two elements of type J, at least one element of type K1 and at least one element of type K2.

3. Apparatus according to one of the claims 1 or 2 comprising 1 to 95 % of elements of type K in the distillation-reaction zone, of one type or another type K1 or K2.

4. Apparatus according to any one of the claims 1 to 3, wherein at least one container chosen from the group formed from among elements of type J and elements of type K has at least one means having the double function of the mechanical reinforcement of said container and maintaining a minimum spacing between said container and at least one of the following elements: the closest container in contact with the end of said means and one of the closest solid surfaces in contact with the end of said means.

5. Apparatus according to any one of the claims 1 to 4, wherein the elements of type J and the elements of type K occupy at least part of the volume of the distillation-reaction zone between said perforated support and a similar perforated device permitting the passage of fluids and whose perforations are sufficiently small to retain said elements between said support and said device.

6. Apparatus according to any one of the claims 1 to 5, wherein the elements of type K have means making it possible to separate them from the elements of type J by the use of magnetic means.

7. Use of an apparatus according to any one of the claims 1 to 6 for performing chemical reactions and the fractionation of the reaction mixture.

8. Use of an apparatus according to any one of the claims 1 to 7 for the production of ethers by the reaction of olefins having 3 to 8 carbon atoms per molecule with alcohols having 1 to 6 carbon atoms per molecule.

9. Process for the preparation of an ether by the reaction of olefins having 3 to 8 carbon atoms per molecule with alcohols having 1 to 6 carbon atoms per molecule, in the presence of an acid catalyst in the form of solid particles, according to which the reaction and the distillation of the products formed during said reaction are carried out concurrently in an apparatus according to any one of the claims 1 to 6 and in which the ether formed is continuously recovered.

**Patentansprüche**

1. Destillations-Reaktionsvorrichtung, wenigstens eine Destillations-Reaktionszone umfassend, wobei diese Zone auf einem den Durchgang der Fluide ermöglichenden perforierten Träger eine Vielzahl von Elementen vom Typ J enthält, die einen Behälter umfassen, der eine Menge an katalytischen festen Partikeln umschließt, der Art, daß der Füllgrad Z1 des Behälters das Verhältnis zwischen dem Volumen Va dieser Partikel, gemessen nach ihrer Kontaktierung mit der Charge unter den Reaktionsbedingungen und dem Innenvolumen Vt des Behälters zwischen 0 und 0,9, untere Grenze ausgeschlossen und obere Grenze eingeschlossen, ist, wobei der Behälter einen Außenmantel hat, der permeabel für die Fluide und impermeabel für diese katalytischen Feststoffpartikel ist, und eine Vielzahl von Elementen vom Typ K, die wenigstens die doppelte Funktion haben, einen bestimmten Füllgrad in der Destillations-Reaktionszone zu sichern sowie einen Destillationseffekt zu sichern, wobei das Element vom Typ K gewählt ist aus der Gruppe, die gebildet wird durch: die Destillationsfüllkörper, welche Elemente vom Typ K1 bilden und die leeren Behälter oder die nicht-leeren Behälter, die einen oder mehrere Füllkörper enthalten, oder nicht-leeren Behälter, welche Elemente vom Typ K2 bilden, wobei die Elemente vom Typ J und vom Typ K, die eine zufällige Stapelung in der Schüttung bilden und das Strömungsverhalten der Fluide ermöglichen über ausreichende mechanische Eigenschaften verfügen, um ohne übermäßige Verformung dem Druck der Gesamtheit der Elemente vom Typ J und vom Typ K, die in der Destillations-Reaktionszone vorhanden sind, widerstehen zu können und die Perforationen dieses perforierten Trägers ausreichend klein sind, um diese Elemente vom Typ J und vom Typ K zurückzuhalten.

2. Vorrichtung nach Anspruch 1, bei der die Destillations-Reaktionszone wenigstens eine der Kombinationen enthält: wenigstens zwei Elemente vom Typ J und wenigstens zwei Elemente vom Typ K1; wenigstens zwei Elemente vom Typ J und wenigstens zwei Elemente vom Typ K2; wenigstens zwei Elemente vom Typ J, wenigstens ein Element vom Typ K1 und wenigstens ein Element vom Typ K2.

3. Vorrichtung nach einem der Ansprüche 1 oder 2, 1 bis 99 % der Elemente vom Typ K in der Destillations-Reaktionszone vom einen oder anderen Typ K1 oder K2 umfassend.

4. Vorrichtung nach einem der Ansprüche 1 bis 3, bei dem wenigstens ein Behälter, gewählt aus der Gruppe, die gebildet wird durch die Elemente vom Typ J und die Elemente vom Typ K wenigstens ein Mittel umfaßt, das die doppelte Funktion der mechanischen Verstärkung dieses Behälters und der Aufrechterhaltung eines minimalen Raums zwischen diesem Behälter und wenigstens einem der folgenden Elemente hat: dem nächsten Behälter in Kontakt mit dem Ende dieses Mittels, einer der festen am weitesten benachbarten Flächen in Kontakt mit dem Ende dieses Mittels.

5. Vorrichtung nach einem der Ansprüche 1 bis 4, bei der die Elemente vom Typ J und die Elemente vom Typ K wenigstens einen Teil des Volumens der Destillations-Reaktionszone einnehmen, der zwischen diesem perforiertem Träger und dieser perforierten ähnlichen Vorrichtung sich befindet und den Durchgang der Fluide ermöglicht und dessen Perforationen ausreichend klein sind, um diese Elemente zwischen dem Träger und dieser Vorrichtung zurückzuhalten.

6. Vorrichtung nach einem der Ansprüche 1 bis 5, bei der die Elemente vom Typ K Mittel umfassen, die es ermöglichen, ihre Trennung von Elementen vom Typ J durch Verwendung magnetischer Mittel durchzuführen.

7. Verwendung einer Vorrichtung nach einem der Ansprüche 1 bis 6 zur Durchführung chemischer Reaktionen und zur Fraktionierung des Reaktionsgemisches.

8. Verwendung einer Vorrichtung nach einem der Ansprüche 1 bis 9 zur Herstellung von Ethern durch Reaktion von Olefinen, die 3 bis 8 Kohlenstoffatome pro Molekül haben, mit Alkoholen, die 1 bis 6 Kohlenstoffatome pro Molekül haben.

9. Verfahren zur Herstellung eines Ethers durch Reaktion von Olefinen, die 3 bis 8 Kohlenstoffatome pro Molekül haben, mit Alkoholen, die 1 bis 6 Kohlenstoffatome pro Molekül haben, in Gegenwart eines sauren Katalysators in Form von Feststoffpartikeln, nach dem man gleichzeitig die Reaktion und die Destillation der während dieser Reaktion gebildeten Produkte in einer Vorrichtung nach einem der Ansprüche 1 bis 6 durchführt und nach dem man kontinuierlich den gebildeten Ether gewinnt.

# FIG.1

FIG.2A

FIG.2C

FIG.5A

FIG.2B

FIG.2D

FIG.2E

FIG.3

FIG.5C

FIG.2F

FIG.5E

FIG.4

FIG.6B

FIG.6A